**Europäisches Patentamt**

**European Patent Office**

**Office européen des brevets**

(19)

(11) Publication number : **0 480 691 A2**

(12) **EUROPEAN PATENT APPLICATION**

(21) Application number : **91309249.0**

(22) Date of filing : **09.10.91**

(51) Int. Cl.⁵ : **A61K 33/24,** A61K 31/60,
A61K 31/44, A61K 31/425,
// (A61K33/24, 31:44,
31:425), (A61K31/60, 31:44,
31:425), (A61K31/44,
31:195), (A61K31/425,
31:195)

(30) Priority : **11.10.90 US 595908**

(43) Date of publication of application :
**15.04.92 Bulletin 92/16**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Berlin, Roger G.**
**519 Mulberry Lane**
**Haverford, PA 19041 (US)**

(74) Representative : **Thompson, John Dr. et al**
**Merck & Co., Inc. European Patent**
**Department Terlings Park Eastwick Road**
**Harlow, Essex CM20 2QR (GB)**

(54) Combination therapy for peptic ulcer treatment.

㋙ Peptic ulcer disease is treated with a combination therapy of famotidine or omeprazole plus a bismuth salt.

EP 0 480 691 A2

## BACKGROUND OF THE INVENTION

In the past, treatment of peptic ulcer disease was based on either neutralization of intragastric acidity with antacids or the inhibition of production of acid secretion by $H_2$-receptor antagonists or by proton pump inhibition among others. Using bismuth salts alone to heal ulcers has been shown to be effective presumably because of its effect on Helicobacter pylori. Relapse of Helicobacter positivity, however, has been a problem as has relapse of ulcer disease in patients treated with antisecretory therapy.

## OBJECTS OF THE INVENTION

It is an object of the present invention to provide compositions and methods of treatment of ulcer disease. Another object is to provide compositions that increase the rate of healing of ulcer disease. A further object is to reduce the relapse and recurrence rate of ulcer disease. Still another object is to provide methods of treating ulcer disease. These and other objects of the present invention will be apparent from the following description.

## SUMMARY OF THE INVENTION

Peptic ulcer disease is treated with a combination therapy of famotidine or omeprazole plus a bismuth salt including bismuth carbonate, bismuth subcarbonate, bismuth subcitrate, bismuth subgallate, bismuth subnitrate and bismuth subsalicylate.

## DETAILED DESCRIPTION

The present invention relates to peptic ulcer disease, and most particularly to duodenal ulcer disease and gastric ulcer disease.

Suspensions of bismuth salts have long been used for gastro-intestinal upsets. While these salts have little or no acid-neutralizing activity, they inhibit pepsin, increase mucus secretion and interact with proteins in the necrotic ulcer crater where they presumably form a barrier to acid diffusion.

Famotidine, an $H_2$ antagonist, inhibits gastric acid secretion elicited by histamine and other $H_2$ agonists and also inhibits acid secretion elicited by gastrin and, to a lesser extent, by muscarinic agonists. The clinical use of famotidine stems largely from its capacity to inhibit gastric acid secretion, especially in patients with peptic ulceration. It is useful to treat duodenal ulcer, gastric ulcer, gastroesophageal reflux disease and maintenance for these conditions and for Zollinger-Ellison syndrome.

Omeprazole, an inhibitor of $H^+,K^+$-ATPase, offers a means to inhibit profoundly acid secretion to any desired level. It is especially useful in patients with gastroesophageal reflux disease and in patients whose peptic ulcer disease is not well controlled by $H_2$ antagonists.

It has now been found that combinations of famotidine or omeprazole with a bismuth salt offer greater initial healing of ulcer disease, or more rapid healing of ulcer disease, or greater initial healing of ulcer disease combined with more rapid healing. These combinations also decrease the frequency of recurrence of ulcer disease over time.

Famotidine is disclosed in U.S. patent 4,283,408. Its efficacy in inhibiting gastric acid and pepsin secretion in man is described by Miwa et al., J. Clin. Pharmacol. Ther. Toxicol. 22, 214 (1984). The results of a clinical trial in Zollinger-Ellison syndrome are described by Howard et al., Gastronenlerology 88, 1026 (1985). Symposia on the pharmacology and clinical efficacy of famotidine are reported in Am. J. Med. 81, Suppl. 4B, 1-64 (1986), and in Scand. J. Gastroenterol. 22, suppl. 134, 1-62 (1987).

Omeprazole is disclosed in U.S. patent 4,255,431. Its pharmacology is described by Muller et al., Arzneimittel-Forsch. 33, 1685 (1983). The results of a clinical trial in Zollinger-Ellison syndrome are described by Lamers et al., N. Engl. J. Med. 310, 758 (1984), and in duodenal ulcer by Lauritsen et al., ibid. 312, 958 (1985), and by Pritchard et al., Brit. Med. J. 290, 601 (1985). A review of pharmacodynamics, pharmacokinetics and therapeutic use is given by Clissold et al., Drugs 32, 15-47 (1986).

The bismuth salts employed in the combinations of the present invention include any bismuth salt useful in treating gastro-intestinal upsets. Examples of such salts are bismuth carbonate, bismuth subcarbonate, bismuth subcitrate, bismuth subgallate, bismuth subnitrate and bismuth subsalicylate.

The combination therapy of the present invention comprises famotidine in a dosage range of from about 10 to about 80 mg/day, typically about 40 mg h.s. or omeprazole in the range of from about 10 to about 80 mg/day, typically about 20 mg a.m., with a bismuth salt useful in treating gastrointestinal upsets in a dosage range of from about 400 to about 600 mg/day in divided doses.

Specific examples of the combination therapy of the present invention follow. The indicated quantity of famotidine or omeprazole is combined with the indicated quantity of any one of the bismuth salts in the same column.

| mg/day | | | | | | | | |
|---|---|---|---|---|---|---|---|---|
| Famotidine | 10 | 20 | 30 | 40 | 50 | 60 | 70 | 80 |
| Bismuth carbonate | 400 | 450 | 500 | 550 | 600 | 400 | 450 | 500 |
| Bismuth subcarbonate | 550 | 600 | 400 | 450 | 500 | 550 | 600 | 400 |
| Bismuth subcitrate | 450 | 500 | 550 | 600 | 400 | 450 | 500 | 550 |
| Bismuth subgallate | 600 | 400 | 450 | 500 | 550 | 600 | 400 | 450 |
| Bismuth subnitrate | 500 | 550 | 600 | 400 | 450 | 500 | 550 | 600 |
| Bismuth subsalicylate | 400 | 450 | 500 | 550 | 600 | 400 | 450 | 500 |

| Omeprazole | 10 | 20 | 30 | 40 | 50 | 60 | 70 | 80 |
|---|---|---|---|---|---|---|---|---|
| Bismuth carbonate | 550 | 600 | 400 | 450 | 500 | 550 | 600 | 400 |
| Bismuth subcarbonate | 450 | 500 | 550 | 600 | 400 | 450 | 500 | 550 |
| Bismuth subcitrate | 600 | 400 | 450 | 500 | 550 | 600 | 400 | 450 |
| Bismuth subgallate | 500 | 550 | 600 | 400 | 450 | 500 | 550 | 600 |
| Bismuth subnitrate | 400 | 450 | 500 | 550 | 600 | 400 | 450 | 500 |
| Bismuth subsalicylate | 550 | 600 | 400 | 450 | 500 | 550 | 600 | 400 |

**Claims**

1. A composition for treating peptic ulcer disease comprising a combination of from about 10 to about 80 mg/day of a gastric acid inhibitor selected from famotidine and omeprazole with from about 400 to about 600 mg/day of a bismuth salt useful in treating gastrointestinal upsets.

2. A composition according to claim 1 wherein the gastric acid inhibitor is famotidine.

3. A composition according to claim 1 wherein the gastric acid inhibitor is omeprazole.

4. A composition according to claim 1 wherein the bismuth salt is bismuth carbonate, bismuth subcarbonate, bismuth subcitrate, bismuth subgallate, bismuth subnitrate or bismuth subsalicylate.

5. The use of a composition according to claim 1 for the manufacture of a medicament for treating peptic ulcer disease.

6. A product containing from about 10 to about 80 mg/day of a gastric acid inhibitor selected from famotidine and omeprazole, and from about 400 to about 600 mg/day of a bismuth salt useful in treating gastrointestinal upsets, for simultaneous, separate or sequential use in the treatment of peptic ulcer disease.